# EUROPEAN PATENT APPLICATION

(11) **EP 4 389 189 A1**
(43) Date of publication of application: **26.06.2024**
(21) Application number: 22858534.5
(22) Date of filing: 19.08.2022
(51) Int. Cl.: A61M 37/00

(54) **HIGH-PERFORMANCE MICRONEEDLE ARRAY**

(30) Priority: 20.08.2021 JP 2021134473
(71) Applicant: Cosmed Pharmaceutical Co., Ltd., Kyoto-shi, Kyoto 601-8014 (JP)
(72) Inventor: QUAN, Ying-shu, Kyoto-shi, Kyoto 601-8014 (JP); SAITO, Mio, Kyoto-shi, Kyoto 601-8014 (JP); YAMASHITA, Hirofumi, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2022/031316
(87) International publication number: WO 2023/022217

(57) **Abstract**

Provided is a microneedle array having high bioavailability and pharmacological availability. A microneedle array including a needle portion and a substrate portion, in which the needle portion contains a valuable material in a base containing a water-soluble polymer as a main component, the substrate portion does not contain the valuable material, and the base of the substrate portion contains a water-insoluble polymer as a main component.

## Description

### TECHNICAL FIELD

The present invention relates to a high-performance microneedle array. Specifically, the present invention relates to the technical field of microneedles that achieve a high valuable material absorption rate and a pharmacological effect.

### BACKGROUND ART

As a method for reliably supplying a medicinal ingredient to a specific site of a skin surface layer and/or a skin stratum corneum, microneedles have been actively studied. For example, in a known self-dissolving microneedle, a target substance is dissolved or dispersed in the entire needle, or dissolved or dispersed in a needle tip end portion. When the valuable material is expensive, if the valuable material is contained in the entire microneedle array, the valuable material contained in a substrate portion of the microneedle array is wasted, and thus the valuable material needs to be contained only in the tip end portion of the microneedle. A base for dissolving or dispersing the target substance is made of a water-soluble polymer substance, and as the base, for example, polysaccharides, proteins, polyvinyl alcohol, carboxyvinyl polymers, polyvinylpyrrolidone, copolymers thereof, and salts thereof have been used. The substrate portion on which the needles stand does not contain the target substance, but a water-soluble polymer similar to the base constituting a needle portion has been used (for example, Patent Documents 1 and 2)

### PRIOR ART DOCUMENT

### PATENT DOCUMENTS

Patent Document 1: Japanese Patent No. 5538897
Patent Document 2: Japanese Patent Laid-open Publication No. 2017-051312

### SUMMARY OF THE INVENTION

### PROBLEM TO BE SOLVED BY THE INVENTION

As the microneedle array, a microneedle having a total length of about 150 to 1500 um has been preferably used from the viewpoint of ease of handling (for example, ease of insertion into the skin). However, when a microneedle of this size is applied to the skin using an applicator, the entire needle portion is inserted into the skin, the water-soluble polymer (base) dissolves in the skin, and the target substance moves to the skin accordingly. At that time, it was observed that the target substance present at a rear end portion of the needle portion diffuses into the skin and at the same time also diffuses into the substrate portion. As a result, a part of the target substance contained in the needle portion is transferred to the substrate portion without diffusing into the skin, and the substrate portion is removed from the skin after administration of the microneedle, and thus is not absorbed into the body and is practically discarded as it is. This loss was found to be one obstacle to the improvement of bioavailability and pharmacological availability.

An object of the present invention is to provide a microneedle array having high bioavailability and pharmacological availability.

### MEANS FOR SOLVING THE PROBLEM

In particular, when the target substance is an expensive proteinaceous drug, even a small amount of loss becomes a big problem in commercialization. Therefore, the present inventors have conducted studies to solve this problem, and completed the invention described in detail below.

The present invention is as follows.
[1] A microneedle array including a needle portion and a substrate portion, in which the needle portion contains a valuable material in a base containing a water-soluble polymer as a main component, the substrate portion does not contain the valuable material, and the base of the substrate portion contains a water-insoluble polymer as a main component.
[2] The microneedle array according to [1], in which the water-insoluble polymer in the substrate portion has an SP value of 9.0 or more.
[3] The microneedle array according to [1] or [2], in which a difference between the SP value of the water-soluble polymer in the needle portion and the SP value of the water-insoluble polymer in the substrate portion is 4 or less.
[4] The microneedle array according to any one of [1] to [3], in which the needle portion has a length of 0.02 to 1.5 mm and has a conical shape, a truncated conical shape, a triangular or polygonal pyramid shape, or a triangular or polygonal frustum shape.
[5] The microneedle array according to any one of [1] to [4], in which the substrate portion has a thickness of 0.01 to 1.0 mm.
[6] The microneedle array according to any one of [1] to [5], in which the water-insoluble polymer is one or more selected from the group consisting of cellulose acetate, an epoxy resin, a maleic anhydride-methyl vinyl ether copolymer, and an acrylic acid-acrylic acid ester copolymer.
[7] The microneedle array according to any one of [1] to [6], in which a tip end portion of the needle portion contains a valuable material, and a root portion of the needle portion does not contain the valuable material and includes a base mainly formed of a water-insoluble polymer.
[8] The microneedle array according to [7], in which a length of the root portion formed of the base containing no valuable material and containing a water-insoluble polymer as a main component is 60% or less of a needle length of the entire needle portion.
[9] A method for manufacturing the microneedle array according to any one of [1] to [8], the method including:
   1) a step of preparing a mold having a concave microneedle pattern by a lithography method or a precision metal working method;
   2) a step of applying an aqueous solution containing a valuable material and a base water-soluble polymer to a surface of the mold and drying the aqueous solution;
   3) a step of applying a base solution formed of an organic solvent of a base containing a water-insoluble polymer as a main component to a surface of a mold after the drying step and drying the base solution; and
   4) a step of taking out the formed microneedle array from the mold.

### EFFECT OF THE INVENTION

According to the present invention, in order to minimize the loss of the target substance, the base of the substrate portion contains a water-insoluble polymer as a main component, whereby the substrate portion does not swell even at the time of administration of the preparation, and thus the target substance can be prevented from diffusing into the substrate portion. As a result, a higher absorption rate of the target substance and a pharmacological effect can be achieved.

### BRIEF DESCRIPTION OF THE DRAWINGS

FIG. 1 is a cross-sectional view illustrating an example of a microneedle array of the present invention.
FIG. 2 is a cross-sectional view illustrating another example of the microneedle array of the present invention.
FIG. 3 is a micrograph of a type 1 microneedle array prepared in Examples 3 and 4.
FIG. 4 is a micrograph of a type 2 microneedle array prepared in Comparative Examples 3 and 4.
FIG. 5 is a micrograph of a type 3 microneedle array prepared in Comparative Example 5.

### MODE FOR CARRYING OUT THE INVENTION

The microneedle array of the present invention includes a needle portion and a substrate portion. The needle portion of the microneedle array of the present invention includes a base formed of an in vivo soluble substance and a target substance held by the base. The microneedle array of the present invention is a needle-like preparation that is used by inserting a needle portion into the skin, and a target substance is absorbed into the body by dissolving a base of the needle portion. In the microneedle array of the present invention, a target substance is held in all or the tip end portion of the needle portion, and the target substance is dissolved or dispersed in a substance obtained by solidifying a base in which the target substance is dissolved or dispersed. A feature of the present invention is that the substrate portion having no target substance includes a base mainly formed of a water-insoluble polymer rather than a water-soluble polymer.

A skin application preparation of this aspect has a needle shape, and is used by being inserted into a body surface such as skin, and the aspect when the microneedle preparation is administered to the skin is analyzed in detail as follows. First, a case where both the needle portion and the substrate portion according to the known technique are made of a water-soluble substance will be described. When the base of the needle portion is dissolved, the target substance is absorbed into the body. However, since the tip end portion of the needle portion reaches a position deep in the skin where the water content in the tissue is high, the base rapidly swells and dissolves in the tissue, and the target substance diffuses into the skin accordingly. When the needle portion is inserted into the skin, the rear end portion of the needle portion is close to the skin surface and swells due to the leachate from the inside of the skin, but the moisture further passes through the rear end of the needle portion and diffuses to the substrate portion to also swell the substrate portion. The target substance dissolved in the rear end part of the needle portion obtains mobility as the base of the needle portion swells, and diffuses into the skin and simultaneously diffuses into the swollen substrate portion. The target substance diffused into the substrate portion does not effectively act on the skin and causes loss of the target substance.

In order to minimize the loss of the target substance, if the substrate portion is made water-insoluble, the substrate portion does not swell even at the time of administration of the preparation, and thus the target substance can be prevented from diffusing into the substrate portion. As a result, a higher absorption rate of the target substance and a pharmacological effect can be achieved.

As a bases for the needle portion and the substrate portion, it is not preferable that the former is a water-soluble polymer and the latter is a water-insoluble polymer from the viewpoint of compatibility between polymers that are different from each other in terms of preparation. The different polymers are generally poorly compatible, thus compromising the mechanical strength of the microneedle array. As a result of studies, the present inventors have found that when the difference in solubility parameter value (hereinafter, SP value) between the water-soluble polymer in the needle portion and the water-insoluble polymer in the substrate portion is 4 or less in order to maintain affinity between both polymers, practically sufficient mechanical strength can be maintained. The difference between the SP value of the water-soluble polymer in the needle portion and the SP value of the water-insoluble polymer in the substrate portion is usually 5 or less, preferably 4 or less, more preferably 3.5 or less, and still more preferably 3 or less. The difference in SP value means a value obtained by subtracting a smaller SP value from a larger SP value between the SP value of the water-soluble polymer in the needle portion and the SP value of the water-insoluble polymer in the substrate portion.

The SP value is a numerical value unique to an organic substance proposed in the 1950s and still widely used as a parameter for quantifying polarities of a polymer and a low molecular weight, and is determined by measurement or calculation from constituent substituents. There are various documents regarding a method of estimating the SP value from the structural formula of the molecule. For example, see Shinichi Ueda et al., Study of Coating Materials No. 152, P. 41-46 Oct. 2010. The SP value of the target is calculated with reference to these documents. Regarding the SP value of the polymer, the SP value of the repeating unit is defined as the SP value of the polymer. The SP value of the copolymer is defined as the SP value of the present copolymer by the mass ratio of the monomers constituting the copolymer.

A main component of the base of the needle portion is a water-soluble polymer. Examples of the water-soluble polymer include proteins such as collagen (or hydrolyzed collagen) and gelatin; polysaccharides such as hyaluronic acid, dextrin, dextran, proteoglycans, and sodium chondroitin sulfate; polymers such as carboxymethyl cellulose and hydroxyethyl cellulose. All of these water-soluble polymers have an SP value of 14.0 or more. In addition, as the water-soluble polymer, at least one selected from the group consisting of synthetic polymers such as polyvinyl alcohol (12.6), carboxyvinyl polymer (14.0), polyvinyl pyrrolidone (14.9), copolymers thereof, and salts thereof can be used. The numbers in parentheses are SP values.

The base of the needle portion may be a water-soluble polymer. The base of the needle portion may contain components other than the water-soluble polymer as long as the needle portion swells and dissolves after being administered to the skin. The content of the water-soluble polymer is preferably 50 mass% or more, more preferably 60 mass% or more, still more preferably 65 mass% or more, even more preferably 70 mass% or more, particularly preferably 80 mass% or more, and most preferably 90 mass% or more as the concentration of the water-soluble polymer in the base of the needle portion. The content of the water-soluble polymer may be 100 mass% or less, less than 100 mass%, 98 mass% or less, or 95 mass% or less as the concentration in the base of the needle portion.

The base of the needle portion of the microneedle array can contain a water-soluble low-molecular compound in addition to the water-soluble polymer. Examples of the water-soluble low molecular compound include a monosaccharide, a disaccharide, or a polyhydric alcohol, and a compound having a molecular weight of 500 or less. Examples of the monosaccharide include glucose and fructose, and examples of the disaccharide include sucrose, lactose, trehalose, and maltose. Examples of the polyhydric alcohol include glycerin, propylene glycol (PG), butylene glycol (BG), polyethylene glycol (PEG) 200, and PEG 400. One or more kinds of these can be used.

The content of the water-soluble low molecular compound is 2 mass% or more and less than 50 mass%, and preferably 2 mass% or more and 35 mass% or less as the concentration in the base of the needle portion.

A main component of the base of the substrate portion is a water-insoluble polymer. The water-insoluble polymer of the base of the substrate portion, which requires affinity with the water-soluble polymer of the needle portion, also requires a high SP value. Specifically, at least one selected from the group consisting of ethyl cellulose (10.3), cellulose acetate (11.0), an epoxy resin (11.2), a maleic anhydride-methyl vinyl ether copolymer (10.0), an acrylic acid-acrylic acid ester copolymer (8.7 to 11.8), and polycyanoacrylate (10.7) can be used as the water-insoluble polymer. Many water-soluble polymers have an SP value of 12.6 or more, and in consideration of affinity with them, the SP value of the water-insoluble polymer of the base of the substrate portion is preferably 9.0 or more, and more preferably 9.5 or more.

As described above, the water-soluble polymer of the needle portion and the water-insoluble polymer of the substrate portion are required to have high affinity and high adhesiveness based thereon. When the adhesiveness of the interface between the two polymers is weak, the interface is peeled off by the impact at the time of skin administration after microneedle molding, the microneedle is broken, and insertion of the needle portion into the skin becomes unstable. The strength of the adhesive strength of the interface can be quantitatively evaluated by measuring the breakage or peeling behavior of the needle by measuring the compressive strength of the molded microneedle.

The base of the substrate portion may be a water-insoluble polymer. The base of the substrate portion may contain other components as long as the substrate portion does not swell or dissolve after being administered to the skin. The content of the water-insoluble polymer is preferably 50 mass% or more, more preferably 60 mass% or more, still more preferably 70 mass% or more, even more preferably 80 mass% or more, and particularly preferably 90 mass% or more as the concentration in the base of the substrate portion. The content of the water-insoluble polymer may be less than 100 mass% or 95 mass% or less as the concentration of the base of the substrate portion.

The microneedle array of the present invention may contain a valuable material such as a cosmetic raw material or a medicinal ingredient, particularly a polymer medicinal ingredient. In the microneedle array of the present invention, it is preferable that the needle portion contains valuable materials and the substrate portion does not contain valuable materials. The content of the valuable material is appropriately 0.001 mass% to 30 mass% with respect to the needle portion mass.

Examples of the valuable materials as raw materials of the cosmetics include skin whitening ingredients such as ascorbyl palmitate, kojic acid, lucinol, tranexamic acid, licorice extract for oil, a vitamin A derivative, a placenta extract, and magnesium adenosine sulfate; anti-wrinkle ingredients such as retinol, retinoic acid, retinol acetate, retinol palmitate, EGF, a cell culture extract, and adenosine; blood circulation promoting ingredients such as α-tocopherol, tocopherol acetate, capsaicin, and vanillylamide nonylate; dietetic ingredients such as raspberry ketone, an evening primrose extract, and a sea weed extract; antimicrobial ingredients such as isopropylmethylphenol, a photosensitive element, and zinc oxide; vitamins such as vitamin D₂, vitamin D₃, and vitamin K.

Examples of the valuable materials as the polymeric medicinal ingredients include physiologically active peptides and derivatives thereof, nucleic acid, oligonucleotide, various antigenic proteins for vaccine applications, bacteria, viral fragments, and antibodies.

Examples of the physiologically active peptides and derivatives thereof include calcitonin, adrenocorticotropic hormone, parathyroid hormone (PTH), hPTH (1 → 34), insulin, exendin and derivatives thereof, secretin, oxytocin, angiotensin, β-endorphin, glucagon, vasopressin, somatostatin, gastrin, luteinizing hormone releasing hormone, enkephalin, neurotensin, atrial natriuretic peptide, growth hormone, growth hormone releasing hormone, FGF, bradykinin, substance P, dynorphin, erythropoietin, thyroid stimulating hormone, prolactin, interferon, interleukin, G-CSF, glutathione peroxidase, superoxide dismutase, desmopressin, somatomedin, endothelin, and salts thereof. Examples of the antigenic protein include an influenza virus antigen, an HBs surface antigen, and an HBe antigen, and examples of the antibody include various polyclonal antibodies and monoclonal antibodies to be subjected to an antibody drug.

The microneedle array of the present invention includes a needle portion and a substrate portion.

The needle portion has a length of 0.02 to 1.5 mm, preferably a length of 0.1 to 1.0 mm. Examples of the shape of the needle portion include a conical shape, a truncated cone shape, a triangular or polygonal pyramid shape, and a triangular or polygonal frustum shape.

The substrate portion has a thickness of 0.01 to 1.0 mm, preferably a thickness of 0.05 to 0.4 mm.

The microneedle array of the present invention may contain valuable materials in the needle portion, but may contain valuable materials in the tip end portion of the needle portion and may not contain valuable materials in the root portion of the needle portion. In this case, the root portion of the needle portion is desirably made of a base containing a water-insoluble polymer as a main component. As the water-insoluble polymer, a polymer similar to the water-insoluble polymer used for the base of the substrate portion can be used.

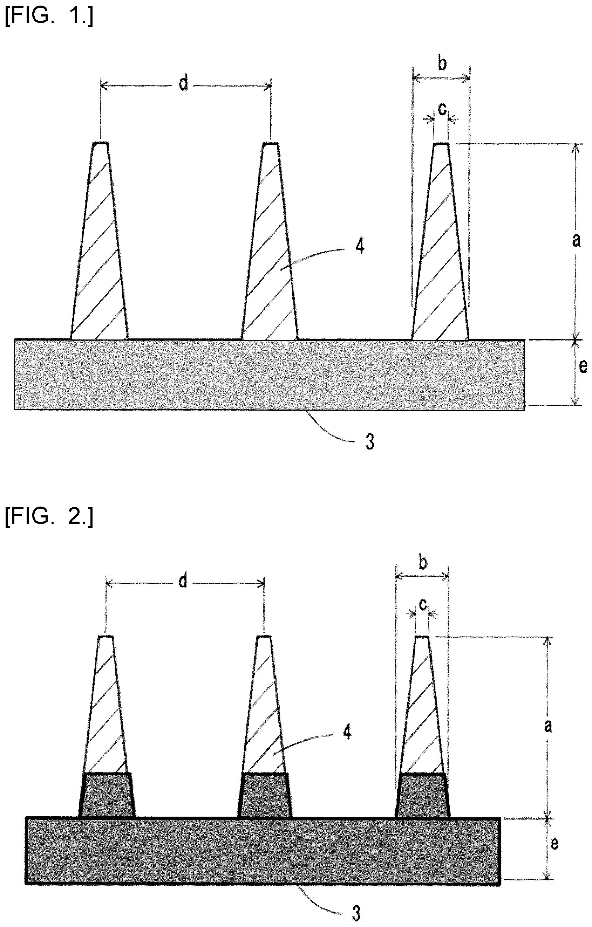

In a case where the needle portion of the microneedle array of the present invention includes a tip end portion and a root portion (refer to, for example, FIG. 2), the length of the tip end portion is 0.01 to 1.4 mm, preferably 0.1 to 1.2 mm. The length of the root portion is 60% or less of the needle length of the entire needle portion, and is 0.05 to 0.7 mm, preferably 0.1 to 0.3 mm.

### Method for manufacturing microneedle array

The method for manufacturing a microneedle array of the present invention includes the following steps 1) to 4).
1) Step of preparing a mold having a concave microneedle pattern by a lithography method or a precision metal working method.
   As the lithography method and the precision metal working method in step 1), methods known in the art can be used.
2) Step of applying an aqueous solution containing a valuable material and a base water-soluble polymer (an aqueous solution containing a valuable material and a water-soluble polymer) to a surface of the mold and drying the aqueous solution.
   In performing step 2), it is effective to promote the filling of the aqueous solution into a mold concave part by pressurization, decompression, centrifugation, or the like. Before the aqueous solution (coating liquid) applied to the mold surface is sufficiently dried, a semi-solid material on the mold surface is scraped off. When the mold surface portion is scraped off in a state where the coating liquid is substantially dried, the proportion of the needle portion containing the valuable material is large, and when the drying time of the coating liquid is short, the resulting microneedle array has a small proportion of the needle portion containing the valuable material.
3) Step of applying a base solution formed of an organic solvent of a base containing a water-insoluble polymer as a main component (a base solution containing a water-insoluble polymer and an organic solvent) to a surface of a mold after the drying step and drying the base solution.
4) Step of taking out the formed microneedle array from the mold.

### EXAMPLES

Hereinafter, the present invention will be described in more detail by the following Examples. These examples are merely examples for specifically describing the present invention, and the scope of the present invention is not limited to these examples.

### Manufacturing of mold

In preparing the microneedle array, first, a microneedle pattern having a predetermined shape was formed by a lithography method of irradiating a photosensitive resin with light, and then a mold in which a microneedle forming concave portion was formed by transferring the microneedle pattern having a predetermined shape by electroforming was manufactured.

The microneedle forming concave portion of the mold have a conical shape with an inlet diameter of 0.2 to 0.5 mm, a bottom diameter of 0.02 to 0.1 mm, and a depth of 0.02 to 1.5 mm, and are arranged in a lattice pattern at intervals of 0.3 to 1.0 mm. The area was a 10 x 10 cm square.

FIG. 1 illustrates an example of a cross-sectional shape of a microneedle array formed by this mold. Each microneedle has a truncated cone shape having a needle portion root diameter (b) of 0.3 mm, a needle portion tip diameter (c) of 0.04 mm, and a needle portion length (a) of 0.8 mm, and is arranged in a lattice pattern at an interval (d) of 0.6 mm. When the microneedle array is formed in a circular shape, about 250 needle portions 4 are included in 1 cm². A substrate portion 3 is formed of a polymer different from that of the needle portion 4, and has a thickness (e) of 0.4 mm. Hereinafter, the microneedle array having this shape is referred to as 800-MN.

FIG. 2 illustrates another example of a cross-sectional shape of a microneedle array formed by this mold. The shape of each microneedle is the same as that of the microneedle array in FIG. 1, but since the root portion of the needle portion 4 in FIG. 2 is formed of the same polymer as the polymer constituting the substrate portion 3, the needle portion tip end portion and the needle portion root portion are formed of different polymers. The length of the needle portion tip end portion containing the valuable material is shorter than 0.8 mm.

### Preparation of microneedle array

Using the manufactured mold, 800-MN microneedle arrays of Examples 1 and 2 and Comparative Examples 1 and 2 having compositions shown in Table 1 were manufactured.

The needle portion was molded using a mold for molding 800-MN. An aqueous solution (solution containing valuable material, base water-soluble polymer, and solvent) as a material of the needle portion shown in Table 1 was prepared, and the aqueous solution was cast into an 800-MN mold. Then, the mold was dried under a pressure of 1.0 KPa for 1 hour and taken out, and the semi-solid on the mold surface was taken out by scraping. A concentrated aqueous solution of the base containing the valuable material remained in the concave portion. Thereafter, a base solution (solution containing a base), which was a material of the substrate portion, was sieved on the mold surface so that the thickness of the substrate portion after drying was 0.4 mm, and the base solution was dried under a pressure of 1.0 KPa for 12 hours. The microneedle array formed on the surface of the mold was peeled off from the mold to take out the microneedle array, and the microneedle array was punched into a circle having a diameter of 10 mm to obtain a microneedle patch. The content of zolmitriptan in Example 2 and Comparative Example 1 was determined from the drug concentration measured by extracting valuable materials from the prepared microneedle patch, and was about 450 µg.

As a reference example, subcutaneous injection of 450 µg of zolmitriptan was also performed, and the blood concentration was measured by blood sampling over time.

**[Table 1]**

| Examples Comparative Examples | Material of needle portion (valuable material, base, and solvent) | | | | Material of substrate portion (base and solvent) | | |
|---|---|---|---|---|---|---|---|
| | Valuable material and concentration in solution | Polymer and concentration in solution | SP value of polymer | Solvent | Polymer and concentration in solution | SP rating of polymer | Solvent |
| Example 1 | Influenza vaccine antigen 1 mass% | Hyaluronic acid 10 mass% | 15 or more | Water | Maleic anhydride -methyl vinyl ether copolymer 30 mass% | 10.0 | Butanone |
| Example 2 | Zolmitriptan 5 mass% | Polyvinylpyrrolidone 20 mass% | 14.9 | Water | Epoxy resin 100 mass% | 11.2 | None |
| Comparative Example 1 | Zolmitriptan 5 mass% | Polyvinylpyrrolidone 20 mass% | 14.9 | Water | Hyaluronic acid 10 mass% | 15 or more | Water |
| Comparative Example 2 | Influenza vaccine antigen 1 mass% | Hyaluronic acid 10 mass% | 15 or more | Water | Polystyrene 10 mass% | 8.5 | Toluene |

### Evaluation

### (1) Moldability

The prepared microneedle patch was microscopically observed, and the moldability of the needle was evaluated.

### (2) Performance evaluation

A microneedle patch (Example 2 and Comparative Example 1) containing zolmitriptan as a valuable material was administered to a Wistar rat (6 to 8 weeks old) by abdomen administration using an applicator, and taken out after 30 minutes. Blood was collected at 0 (before administration), 15, 30, 60, 120, 180, 300, and 420 minutes later, the blood concentration of zolmitriptan was measured, the blood concentration-time curve was determined, and the bioavailability (BA) was calculated. For comparison, blood concentration-time curves were obtained also for subcutaneous injection administration and oral administration. The total test was performed with N = 3, and the average value thereof was obtained.

### Results

The results are shown in Table 2.

**[Table 2]**

| Examples and Comparative Examples | Moldability | Performance evaluation |
|---|---|---|
| Example 1 | The moldability of the needle was good, and a microneedle patch in which needles were formed in an amount of 90% or more of the theoretical amount was obtained. | - |
| Example 2 | Same as above | The BA value obtained from the blood concentration curve at 0 to 420 minutes was 100.3% compared to the BA value determined from subcutaneous injection. |
| Comparative Example 1 | Same as above | The BA value obtained from the blood concentration curve at 0 to 420 minutes was 43.9% as compared with the BA value determined from subcutaneous injection. |
| Comparative Example 2 | A microneedle patch in which the needle had poor needle moldability and the needle was formed in an amount of 20% or less of the theoretical amount was obtained. | - |
| Comparative Example 3 (oral administration) | - | The BA value obtained from the blood concentration curve at 0 to 420 minutes was 14.3% as compared with the BA value determined from subcutaneous injection. |

The reason why the BA value is low in Comparative Example 1 is considered to be that the target substance dissolved in the rear end portion of the needle is diffused into the skin with mobility and at the same time diffused into the swollen substrate portion as the base of the needle portion and the base of the substrate portion are swollen by body moisture. As a result of extracting and quantifying the drug separately transferred to the substrate portion, it was 28%.

In Comparative Example 2, since there is a large difference in polarity between the polymer of the base of the needle portion and the polymer of the base of the substrate portion, and compatibility is small, the microneedle array was broken from a bonded portion when taken out from the mold.

### Preparation 2 of microneedle array

In preparing the microneedle array, first, a microneedle pattern having a predetermined shape was formed by a lithography method of irradiating a photosensitive resin with light, and then a mold in which a microneedle forming concave portion was formed by transferring the microneedle pattern having a predetermined shape by electroforming was manufactured.

The microneedle forming concave portion of the mold have a conical shape with an inlet diameter of 0.2 to 0.5 mm, a bottom diameter of 0.02 to 0.1 mm, and a depth of 0.02 to 1.5 mm, and are arranged in a lattice pattern at intervals of 0.3 to 1.0 mm. The area was a 10 x 10 cm square.

In this experiment, a plastic mold having a needle length of 0.9 mm, a needle interval of 0.75 mm, a needle root diameter of 0.3 mm, and a recess having a conical needle shape was used.

A polyvinyl alcohol or hyaluronic acid aqueous solution containing 0.05 mass% of a red pigment (New coccine, manufactured by FUJIFILM Wako Pure Chemical Corporation) was filled in the recess of the mold, and dried. Subsequently, the dried mold was filled with various polymers for forming the substrate portion as a solution of water or an organic solvent, and then centrifuged for 20 minutes for integration with the needle portion. Thereafter, drying was performed for 12 hours, the microneedle array was taken out from the mold, and the state of the microneedle array was observed with a microscope. The characteristics of the prepared microneedle array (Examples 3 and 4 and Comparative Examples 3 to 5) are shown in Table 3. The solvent of the water-soluble polymer was water. The thickness of the substrate portion in the obtained microneedle array was 0.3 to 0.5 mm.

**[Table 3]**

| Examples Comparative Examples | Needle portion | | Substrate portion | | Difference in SP value | Microneedle array type |
|---|---|---|---|---|---|---|
| | Polymer | Presence or absence of water solubility of polymer and SP value | Polymer | Presence or absence of water solubility of polymer and SP value (solvent) | | |
| Example 3 | Polyvinyl alcohol | Water solubility SP: 12.6 | Ethyl cellulose | Water insoluble SP: 10.3 (Ethanol) | 2.3 | Type 1 |
| Example 4 | Same as above | Same as above | Polycyanoacrylate | Water insoluble SP: 10.7 (Monomer casting) | 1.9 | Type 1 |
| Comparative Example 3 | Same as above | Same as above | Hyaluronic acid | Water solubility SP: 15.6 | 3.0 | Type 2 |
| Comparative Example 4 | Hyaluronic acid | Water solubility SP: 15.6 | Polyvinylpyrrolidone | Water solubility SP: 14.9 | 0. 7 | Type 2 |
| Comparative Example 5 | Same as above | Water solubility SP: 15.6 | Polystyrene | Water insoluble SP: 8.5 (Toluene) | 7.1 | Type 3 |

Although different microneedle arrays were prepared using polymers of various substrate portions, the obtained microneedle arrays were roughly classified into the following three types.

### Type 1

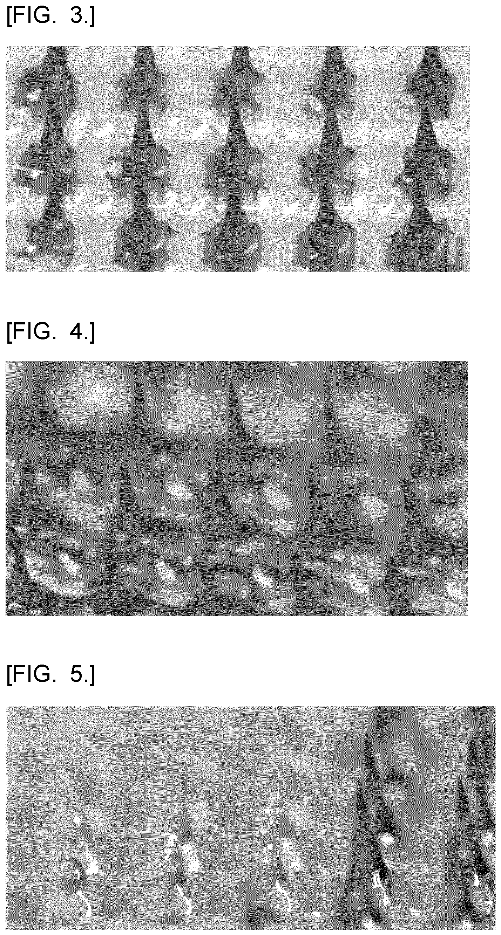

As illustrated in FIG. 3, the needle portion and the substrate portion are completely integrated. The pigment in the needle portion does not diffuse to the substrate portion and remains in the needle portion. The gist of the present invention is realized.

### Type 2

As illustrated in FIG. 4, the needle portion and the substrate portion are completely integrated. However, the dye of the needle portion is diffused to the substrate portion. In this case, when the drug filled in the needle portion is water-soluble, the drug diffuses into the substrate portion formed of the water-soluble polymer in the drying step of the microneedle array, and as a result, the drug concentration in the needle portion decreases, which is disadvantageous.

### Type 3

As illustrated in FIG. 5, when the microneedle array is taken out from the mold, the needle portion remains in the mold, and complete integration with the substrate portion is not realized (left half part in FIG. 5). This is because the difference in polarity between the polymers of the needle portion and the substrate portion is large and the affinity between the polymers is small.

### DESCRIPTION OF REFERENCE SYMBOLS

- 3: Substrate portion
- 4: Needle portion

## Claims

1. A microneedle array including a needle portion and a substrate portion, wherein the needle portion contains a valuable material in a base containing a water-soluble polymer as a main component, the substrate portion does not contain the valuable material, and the base of the substrate portion contains a water-insoluble polymer as a main component.

2. The microneedle array according to claim 1, wherein the water-insoluble polymer in the substrate portion has an SP value of 9.0 or more.

3. The microneedle array according to claim 1 or 2, wherein a difference between the SP value of the water-soluble polymer in the needle portion and the SP value of the water-insoluble polymer in the substrate portion is 4 or less.

4. The microneedle array according to any one of claims 1 to 3, wherein the needle portion has a length of 0.02 to 1.5 mm and has a conical shape, a truncated conical shape, a triangular or polygonal pyramid shape, or a triangular or polygonal frustum shape.

5. The microneedle array according to any one of claims 1 to 4, wherein the substrate portion has a thickness of 0.01 to 1.0 mm.

6. The microneedle array according to any one of claims 1 to 5, wherein the water-insoluble polymer is one or more selected from the group consisting of cellulose acetate, an epoxy resin, a maleic anhydride-methyl vinyl ether copolymer, and an acrylic acid-acrylic acid ester copolymer.

7. The microneedle array according to any one of claims 1 to 6, wherein a tip end portion of the needle portion contains a valuable material, and a root portion of the needle portion does not contain the valuable material and includes a base mainly formed of a water-insoluble polymer.

8. The microneedle array according to claim 7, wherein a length of the root portion formed of the base containing no valuable material and containing a water-insoluble polymer as a main component is 60% or less of a needle length of the entire needle portion.

9. A method for manufacturing the microneedle array according to any one of claims 1 to 8, the method comprising:
1) a step of preparing a mold having a concave microneedle pattern by a lithography method or a precision metal working method;
2) a step of applying an aqueous solution containing a valuable material and a base water-soluble polymer to a surface of the mold and drying the aqueous solution;
3) a step of applying a base solution formed of an organic solvent of a base containing a water-insoluble polymer as a main component to a surface of a mold after the drying step and drying the base solution; and
4) a step of taking out the formed microneedle array from the mold.
